# EUROPÄISCHE PATENTSCHRIFT

(11) **EP 2 649 958 B1**
(45) Veröffentlichungstag und Bekanntmachung des Hinweises auf die Patenterteilung: **29.03.2017**
(21) Anmeldenummer: 13176165.2
(22) Anmeldetag: 16.06.2010
(51) Int. Cl.: A61B 18/14, A61N 1/06, A61B 18/00, A61B 18/02

(54) **Elektrochirurgisches Instrument**
Electrosurgical instrument
Instrument électrochirurgical

(30) Priorität: 07.07.2009 DE 102009032065; 05.10.2009 DE 102009048312
(43) Veröffentlichungstag der Anmeldung: 16.10.2013
(62) Teilanmeldung aus: 10724325.5
(73) Patentinhaber: Erbe Elektromedizin GmbH, 72072 Tübingen (DE)
(72) Erfinder: Besch, Hansjörg Björn, 72810 Gomaringen (DE); Strehle, Britta, 72072 Tübingen (DE)
(74) Vertreter: Rüger, Barthelt & Abel

(56) Entgegenhaltungen:
- EP-A2- 1 568 332
- DE-A1-102005 023 303
- GB-A- 2 308 979
- US-A- 5 697 927
- US-A1- 2006 217 791

## Beschreibung

Die Erfindung betrifft eine Ablationssonde gemäß dem Anspruch 1.

In der HF-Chirurgie (Hochfrequenzchirurgie) ist es bekannt, Gewebe gezielt durch die Applikation eines HF-Stroms zu devitalisieren. Ein entsprechender Vorgang kann durch ein monopolares oder bipolares Instrument vorgenommen werden. Bei der monopolaren Technik führt der Strompfad üblicherweise vom elektrochirurgischen Instrument des zu behandelnden Gewebes zur Neutralelektrode. Andererseits können bipolare Instrumente verwendet werden, die mit zwei voneinander elektrisch isolierten Abschnitten ausgebildet sind. Der Stromweg verläuft in diesem Fall von einem ersten Abschnitt des elektrochirurgischen Instruments über das zu behandelnde Gewebe zu einem zweiten Abschnitt des elektrochirurgischen Instruments.

Es sind Kryosonden bekannt, die Distal- und Proximalelektroden aufweisen, um einen entsprechenden Strom zu applizieren. Diese Kryosonden verfügen über eine Kühlvorrichtung, die es ermöglicht, das unmittelbar an dem Instrument anliegende Gewebe zu kühlen. Somit kann eine ungewollte Karbonisation des umliegenden Gewebes verhindert werden, so dass das Instrument zu einer kontrollierteren und möglicherweise grossflächigeren Devitalisierung eingesetzt werden kann. Des Weiteren lässt sich durch den gezielten Einsatz der Kühlvorrichtung die Wärmeverteilung entlang des Instruments steuern und somit der Devitalisierungsbereich einstellen.

Ein entsprechender Kühlvorgang kann beispielsweise durch das gezielte Einsetzen des Joule-Thomson-Effekts bewerkstelligt werden. Hierbei erfährt ein Fluid, insbesondere ein Gas, durch Drosselung (Druckänderung) eine Temperaturveränderung.

Es sind elektrochirurgische Instrumente bekannt, bei denen an der Außenseite eines Sondenschlauchs eine Distalelektrode und eine Proximalelektrode angeordnet sind. Im Lumen des Sondenschlauchs befindet sich ein Fluid- oder Gaskanal, der ein Kühlfluid am distalen Ende des Sondenschlauchs in einer Expansionskammer bereitstellt. Das Lumen des Sondenschlauchs wird dazu verwendet, das expandierte Fluid abzuführen. Zur elektrischen Verbindung der Distalelektrode und der Proximalelektrode mit einem HF-Generator dienen zum einen der elektrisch leitfähige Gaskanal sowie eine in dem Lumen getrennt geführte und gegenüber dem Gaskanal isolierte Leitung. Die elektrische Verbindung der Proximalelektrode mit dieser Leitung gestaltet sich als schwierig. So werden Öffnungen in dem Sondenschlauch vorgesehen, um eine entsprechende Kontak-tierung vorzunehmen. Durch diese Öffnungen kann das Fluid das im Lumen geführt wird, in das Organ eintreten und hier zu einer Schädigung führen. Der Fertigungsaufwand zur Herstellung derartiger Sonden, insbesondere entsprechender Bohrungen, ist hoch. Des Weiteren müssen die Sonden so dimensioniert werden, dass das Lumen zur Aufnahme der zusätzlichen Leitung ausreichend Platz bereitstellt. Für minimalinvasive Eingriffe ist es jedoch notwendig, dass der Durchmesser der Sonde sehr gering ist.

Aus der EP 1 902 683 A1 ist ein Katheter zur Applikation eines Stroms bekannt. Der Katheter umfasst hierfür mehrere Elektroden, die über Leiterbahnen innerhalb eines Schlauchs des Katheters mit einer geeigneten Spannung versorgt werden. Die Leiterbahnen befinden sich innerhalb der Außenwand des Schlauchs und sind durch diese elektrisch gegeneinander isoliert. Außerdem ist aus der US 2006/01217791 A1 ein Katheder mit einem schlauchartigen Grundkörper bekannt, der eine Anzahl von Lumen enthält, über die beispielsweise Fluide zu- oder abgeführt werden können. An der Außenseite des aus einem Kunststoff bestehenden schlauchartigen Grundkörpers sind zwei Leitungsfolien helixartig aufgebracht. Sie enden in einer oder mehreren Elektroden, die freiliegen, um mit einem medizinischen Instrument oder einem Nervenende in Berührung gebracht zu werden.

Die EP 1 568 332 zeigt ein chirurgisches Instrument zur elektrothermischen Koagulation mit einer Distalelektrode und einer Proximalelektrode. Das Instrument weist auch einen zentralen Hohlkanal auf, durch den ein Spülschlauch hindurchläuft, der Flüssigkeit am distalen Ende abgibt zur Kühlung des Instruments. Die Distal- bzw. Proximalelektrode ist durch eine im Hohlkanal verlaufende Anschlussleitung an einen HF-Generator anschliessbar.

Ausgehend von diesem Stand der Technik ist es Aufgabe der vorliegenden Erfindung, ein elektrochirurgisches Gerät der eingangs genannten Art bereitzustellen, das sicher ist, geeignete Abmessungen aufweist und sich einfach und effizient herstellen lässt.

Diese Aufgabe wird vorrichtungsseitig durch eine Ablationssonde gemäß dem Anspruch 1 gelöst.

Ein besonderer Vorteil der vorliegenden Erfindung besteht also darin, dass eine elektrisch leitfähige Außenhaut, also die Leitungsfolien des Sondenschlauchs, als elektrische Leitung oder als Leiterbahn benutzt wird. Somit kann die elektrische Leitung für die Elektroden bereitgestellt werden, ohne dass dies bei der Dimensionierung des Sondenschlauchs berücksichtigt werden müsste. Das Vorsehen der Leitungsfolie verursacht nur eine geringfügige Aufdickung der Wandstärke, so dass relativ kleine Sonden entwickelt werden können. Gegenüber dem bekannten Stand der Technik ist es nicht notwendig, den Sondenschlauch zu perforieren, um eine entsprechende Leitung herzustellen. Somit gestaltet sich auch der Herstellungsprozess eines elektrochirurgischen Instruments als relativ einfach. Das transportierte Fluid kann ein Gas, eine Flüssigkeit oder ein Gas-Flüssigkeits-Gemisch sein.

Die Leitungsfolie kann eine Kupferfolie, Goldfolie oder Silberfolie sein. Unter dem Begriff der Kupferfolie bzw. Goldfolie bzw. Silberfolie können sämtliche geeignete Folien aus einer Kupferlegierung bzw. Goldlegierung bzw. Silberlegierung fallen. Durch das Verwenden der Kupferfolie ist es möglich, einen geeigneten Leiter mit geringer Wandstärke herzustellen. Kupfer hat auch einen niedrigen spezifischen Widerstand.

Die Leitungsfolie ist in einer schraubenförmigen Struktur entlang des Sondenschlauchs angeordnet sein. Diese helixartige Wicklung der Folie bewirkt, dass die nötige Flexibilität des Instruments, insbesondere des Sondenschlauchs, nur geringfügig beeinflusst wird. Dies sorgt dafür, dass das elektrochirurgische Instrument ausreichend navigationsfähig bleibt, so dass es problemlos innerhalb des Arbeitskanals eines Endoskops bewegt werden kann. Theoretisch wäre es zwar denkbar, die Leitungsfolien einseitig in Form einer direkten Leitungsbahn entlang der Längsrichtung des Sondenschlauchs zu führen oder eine zickzackförmige Folienbahn an einer Seite des Sondenschlauchs anzubringen, jedoch würde dies auch eine einseitige Änderung der Flexibilität und/oder Elastizität des Schlauches verursachen. Dies würde die Navigationsfähigkeit negativ beeinflussen.

Theoretisch könnten die einzelnen benachbarten Bahnen der Leitungsfolie zumindest abschnittsweise überlappend entlang der Längsrichtung des Sondenschlauchs angeordnet sein. Somit würde sich eine im Wesentlichen schlauchförmige Leitungsbahn ausbilden, die die Flexibilität des Sondenschlauchs nur geringfügig beeinflusst und nur einen geringen induktiven Widerstand aufweist. Vorzugsweise werden die benachbarten Bahnen der Leitungsfolie in Längsrichtung des Sondenschlauchs voneinander beabstandet angeordnet, so dass es zu keinen Überlappungen kommt. Die Überlappungen stellen nämlich eine zusätzliche Aufdickung bezüglich des Durchmessers des elektrochirurgischen Instruments dar. Der induktive Widerstand ist aufgrund der Breite der Bahnen der Leitungsfolie tolerierbar.

Das Instrument kann eine Isolatorschicht umfassen, die zur Fixierung der Leitungsfolie diese zumindest abschnittsweise überdeckt. Vorzugsweise ergibt sich also im Querschnitt eine Anordnung, die sich vom Inneren des Sondenschlauchs nach außen wie folgt gestaltet: Wand des Sondenschlauchs, Leitungsfolie, Isolatorschicht. Die Isolatorschicht kann sehr dünnwandig ausgestaltet sein und isoliert die durch die Leitungsfolie erzeugte Leiterbahn gegenüber dem Außenbereich. Des Weiteren schützt sie die Leitungsfolie vor äußeren Einflüssen und kann diese in Position halten. Die Isolatorschicht kann aus einem Material hergestellt werden, das die Flexibilität des Sondenschlauchs und somit des elektrochirurgischen Instruments nur geringfügig beeinflusst.

Die Distalelektrode und die Proximalelektrode werden durch jeweils eine Leitungsfolie versorgt. Somit kann die elektrische Verbindung zwischen der Leitungsfolie und der zugehörigen Elektrode denkbar einfach ausgebildet sein. Ein Löten ist an dieser Stelle nicht notwendig. Somit ist die Verbindungstechnik rein raumtauglich. Im Proximalbereich des Sondenschlauchs können entsprechende Anschlüsse auch durch eine mechanische Verbindungstechnik hergestellt werden. Beispielsweise kann die Kontaktierung durch ein Crimpen erfolgen.

Ein beispielhaftes elektrochirurgisches Instrument kann eine Kryosonde mit einem Fluidkanal für ein Kühlfluid (z.B. einem Gaskanal) sein, wobei der Fluidkanal ein elektrischer Leiter ist und zur Bereitstellung der HF-Spannung mit der mindestens einen Elektrode verbunden ist. Das elektrochirurgische Instrument kann also eine Kryosonde sein, die vorzugsweise eine in einem Lumen des Sondenschlauchs geführten Gaskanal umfasst. Das über den Fluidkanal eingebrachte Fluid kann aus einem Distalbereich der Sonde über das Lumen abgeführt werden. Zum Beispiel ist der Fluidkanal zumindest abschnittsweise aus einem elektrisch leitfähigen Material hergestellt und dient somit ebenfalls als Leiterbahn. Diese Leiterbahn kann dazu genutzt werden, eine weitere Elektrode, vorzugsweise die Distalelektrode, mit einem HF-Generator zu verbinden.

Weitere vorteilhafte Ausführungsformen ergeben sich anhand der Unteransprüche.

Nachfolgend wird die Erfindung anhand von einigen weiteren Ausführungsbeispielen beschrieben, die mittels Abbildungen näher erläutert werden. Hierbei zeigen:
Fig. 1 eine schematische Darstellung eines elektrochirurgischen Geräts mit Versorgungseinrichtung und Ablationssonde; und
Fig. 2 einen Schnitt durch die Ablationssonde nach Fig. 1.

In der nachfolgenden Beschreibung werden für gleiche und gleich wirkende Teile dieselben Bezugsziffern verwendet.

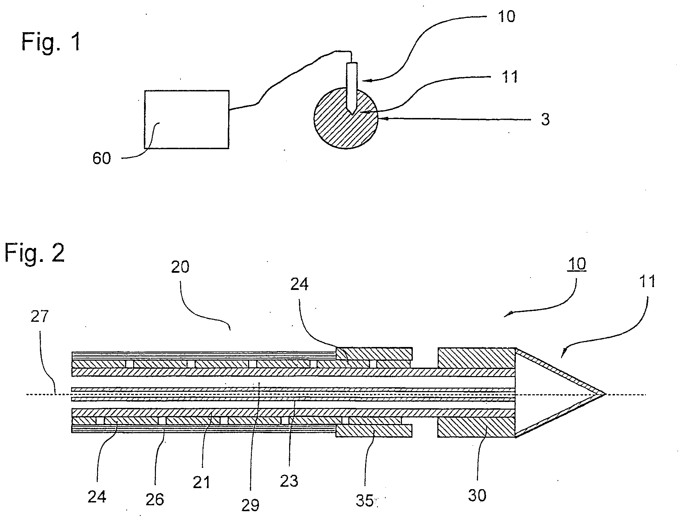

Fig. 1 zeigt ein HF-Chirurgiegerät zur Devitalisierung von Gewebe. Eine Ablationssonde 10 ist über einen Versorgungsschlauch und mehrere Kabel mit einer Versorgungseinrichtung 60 verbunden. Die Ablationssonde 10 ist in ein Gewebe 3 eingeführt, das mittels eines HF-Stroms devitalisiert werden soll, der durch die Ablationssonde appliziert wird. Hierfür sind an
der Ablationssonde 10 angeordnete Elektroden, nämlich eine Distalelektrode 30 und eine Proximalelektrode 35, mit einem HF-Generator in der Versorgungseinrichtung 60 verbunden. Um das Gewebe 3 vollständig zu devitalisieren, ist es notwendig, die Ablationssonde 10 zumindest abschnittsweise zu kühlen, um eine Karbonisation des die Ablationssonde 10 kontaktierenden Gewebes zu vermeiden. Daher umfasst die erfindungsgemäße Ablationssonde 10 eine Kühleinrichtung, die durch die Versorgungseinrichtung 60 gespeist wird.

Bei der Fig. 1 handelt es sich um eine schematische Darstellung, die die Wirkung der erfindungsgemäßen Ablationssonde 10 verdeutlichen soll. Tatsächlich werden derartige Sonden häufig über Endoskope an das Zielgewebe herangeführt, um einen minimalinvasiven Eingriff vorzunehmen. Hierfür ist es notwendig, den Durchmesser der Ablationssonde 10 so gering wie möglich zu halten. Des Weiteren muss die Ablationssonde 10 flexibel sein, um eine Devitalisierung auch an Orten vornehmen zu können, die nur schwer zugänglich sind. Bezüglich der Flexibilität sei noch angemerkt, dass Sonden, die sich in eine Richtung besser biegen lassen als in eine andere, von dem behandelnden Arzt häufig als ungeeignet empfunden werden, da es schwierig ist, derartige Ablationssonden 10 im Arbeitskanal zu führen.

Die erfindungsgemäße Ablationssonde 10 umfasst einen Sondenschlauch 21 (vgl. Fig. 2) mit einem proximalen und einem distalen Ende. Am distalen Ende des Sondenschlauchs 21 ist eine Sondenspitze 11 vorgesehen, die die Ablationssonde 10 abschließt. Am proximalen Ende des Sondenschlauchs 21 kann ein Handgriff zur Führung der Ablationssonde 10 und Anschlüsse zur Verbindung der Ablationssonde 10 mit der Versorgungseinrichtung 60 vorgesehen sein (in Fig. 1 nicht gezeigt).

Der Sondenschlauch 21 definiert eine theoretische Längsachse 27 der Ablationssonde 10, die sich vom proximalen zum distalen Ende der Ablationssonde 10 erstreckt.

Im Inneren des Sondenschlauchs 21, also in einem Lumen 29, befindet sich ein flexibler Gaskanal 23, der vom proximalen Ende der Ablationssonde 10 bis zur Sondenspitze 11 geführt ist. Über diesen Gaskanal 23 wird ein Fluid, vorzugsweise Lachgas, in die Ablationssonde 10 eingebracht. Das Fluid expandiert an einem distalen Ende des Gaskanals und entzieht der Ablationssonde 10 Wärmeenergie. Es kommt zu einer Abkühlung der Ablationssonde 10. Das expandierte Fluid wird über das Lumen 29 abgeführt und entsorgt. Somit sind der Sondenschlauch 21, der Gaskanal 23 und die Sondenspitze 11, die als Expansionskammer dient, ein Teil der Kühleinrichtung der Ablationssonde 10.

Am distalen Ende des Sondenschlauchs 21 in unmittelbarer Nachbarschaft zur Sondenspitze 11 befindet sich die Distalelektrode 30 , die den Sondenschlauch 21 ringförmig umschließt. Die Distalelektrode 30 steht in elektrischer Verbindung mit dem Gaskanal 23 , der aus einem elektrisch leitfähigen Material ausgebildet ist. Somit stellt der Gaskanal 23 zumindest einen Teil einer ersten Leiterbahn oder Leitung zur Versorgung der Distalelektrode 30 aus. Die Verbindung zwischen Distalelektrode 30 und Gaskanal 23 ist in der Fig. 2 nicht gezeigt. Beispielsweise kann die Distalelektrode 30 die vorzugsweise elektrisch leitfähige Sondenspitze 11 unmittelbar kontaktieren, die wiederum die Distalelektrode 30 kontaktiert oder ein Teil dieser ist.

Eine zweite Leiterbahn oder elektrische Leitung zur Versorgung der Proximalelektrode 35 wird durch eine Kupferfolie 24 ausgebildet. Auch bei der Proximalelektrode 35 handelt es sich um eine Ringelektrode, die den Sondenschlauch 21 umschließt und vorzugsweise koaxial zur Längsachse 27 angeordnet ist. Die Proximalelektrode 35 ist gegenüber der Distalelektrode 30 in proximaler Richtung versetzt und von der Distalelektrode 30 ausreichend beabstandet, um eine elektrische Isolationslücke bereitzustellen. Diese Lücke kann mit einem Isolator aufgefüllt werden.

Die Kupferfolie 24 ist unmittelbar auf den Sondenschlauch 21 aufgebracht und hat eine helixartige Wicklung, wobei sich die Kupferfolie 24 vom proximalen Ende des Sondenschlauchs bis unterhalb der Proximalelektrode 35 erstreckt. Die einzelnen Wicklungen um den Sondenschlauch 21 werden als Bahnen bezeichnet, wobei die einzelnen Bahnen in Längsrichtungen einen im Wesentlichen gleichen Abstand aufweisen. Theoretisch wäre es möglich, die Abstände der einzelnen Bahnen zueinander zu variieren.

Die Helixstruktur oder helixartige Anordnung der Kupferfolie 24 hat eine konstante Steigung. Die Proximalelektrode 35 ist unmittelbar auf die Kupferfolie 24 aufgebracht und kontaktiert diese somit. Der elektrische Kontakt zwischen Kupferfolie 24 und Proximalelektrode 35 ist also sichergestellt.

Vorzugsweise umfasst die Ablationssonde 10 eine Außenisolierung 26, die auf die Kupferfolie 24 aufgebracht ist. Diese Außenisolierung 26 erstreckt sich vom proximalen Ende der Ablationssonde 10 bis zur Proximalelektrode 35. Die elektrische Kontaktierung der Kupferfolie 24 am proximalen Ende kann durch ein Crimpen erfolgen.

Der Sondenschlauch 21, die Kupferfolie 24, die Außenisolierung 26, die Distalelektrode 30 und die Proximalelektrode 35 bilden einen Sondenkörper 20.

Die Helixstruktur der Kupferfolie 24 ist ein Band, das sich mit konstanter Steigung entlang des Sondenschlauchs 21 windet. Die Wicklung, sowie die Außenisolierung 27 können derart ausgebildet sein, dass sie die Stabilität der Ablationssonde 10 erhöhen, wobei eine nötige Flexibilität erhalten bleibt.

Die beschriebene Kontaktierung zwischen Kupferfolie 24 und Proximalelektrode 35 lässt sich auch in Hinblick auf den Fertigungsprozess einfach herstellen. Diese vorgeschlagene elektrische Verbindung ist sicher. Da der Leiter oder die Leiterbahn an der Außenseite des Sondenschlauchs 21 geführt ist, müssen keine Durchführungen oder Bohrungen am Sondenschlauch 21 vorgesehen werden. Die Gasdichte des Sondenschlauchs 21 bleibt erhalten.

Im vorab beschriebenen Beispiel wurde ein bipolares elektrochirurgisches Instrument beschrieben, das mindestens zwei Elektroden aufweist. Für den Fachmann sollte es offensichtlich sein, dass die erfindungsgemäße Kontaktierung der Proximalelektrode 35 auch bei monopolaren Instrumenten sinnvoll eingesetzt werden kann. Auch sollte es klar sein, dass an Stelle der beschriebenen einen Kupferfolie 24 mehrere Kupferfolien parallel zueinander angeordnet werden können, um mehrere elektrisch voneinander isolierte Elektroden zu kontaktieren. Erfindungsgemäß sind die Distalelektrode 30 und die Proximalelektrode 35 mittels entsprechender Kupferfolien 24 versorgt. In diesem Fall könnte auf den elektrisch leitfähigen Gaskanal 23 verzichtet werden.

Im vorab beschriebenen Ausführungsbeispiel sind die Bahnen der Kupferfolie 24 parallel zueinander angeordnet. Es liegt keine Überlappung der Kupferfolie 24 vor.

Durch den engen Abstand der Wicklungen und die Breite der Kupferfolie 24 wirkt die Kupferfolie 24 fast wie ein Rohr und die entsprechenden induktiven Widerstände sind gering. Es wäre jedoch auch möglich, die Kupferfolie 24 überlappend anzuordnen, um den induktiven Widerstand zu verringern.

Die Ablationssonde 10 aus Fig. 2 weist zwei Ringelektroden auf, die den Sondenschlauch 21 umschließen. Zum Beispiel können jedoch auch beliebige andere Elektroden entlang des Sondenkörpers 20 der Ablationssonde 10 vorgesehen sein. Beispielsweise könnten die Ringelektroden abschnittsweise unterbrochen sein oder durch Plattenelektroden, die sich beispielsweise entlang der Längsachse 27 erstrecken, ersetzt werden.

Auch wenn vorhergehend eine Ablationssonde 10 beschrieben wurde, bei der ein Gas über einen Gaskanal 23 in den distalen Bereich der Ablationssonde 10 transportiert wird, um dieses dort zu expandieren, so ist die vorliegende Erfindung nicht auf diese spezielle Ausführungsform beschränkt. Die Erfindung lässt sich bei jeglicher Art von Schläuchen anwenden, wobei es irrelevant ist, ob diese zum Transport eines Gases, einer Flüssigkeit oder eines entsprechenden Gemisches verwendet werden.

### Bezugszeichenliste

- 3 :: Gewebe
- 10 :: Ablationssonde
- 11 :: Sondenspitze
- 20 :: Sondenkörper
- 21 :: Sondenschlauch
- 23 :: Gaskanal
- 24 :: Kupferfolie
- 26 :: Außenisolierung
- 27 :: Längsachse
- 29 :: Lumen
- 30 :: Distalelektrode
- 35 :: Proximalelektrode
- 60 :: Versorgungseinrichtung

## Patentansprüche

1. Ablationssonde (10), umfassend:
- einen Sondenschlauch (21) zum Transport von Fluid;
- eine Distalelektrode (30) und eine Proximalelektrode (35), die zur Devitalisierung von Gewebe mittels HF-Stroms mit einer einen HF-Generator enthaltenden Versorgungseinrichtung (60) verbunden sind; und
- Leitungsfolien (24), die entlang einer Längsachse (27) des Sondenschlauchs (21) parallel zueinander und in einer schraubenförmigen Struktur angeordnet sind, wobei die Distalelektrode und die Proximalelektrode elektrisch voneinander isoliert sind und durch jeweils eine Leitungsfolie (24) versorgt werden, wobei die Ablationssonde (10) eine Kühleinrichtung zur zumindest abschnittsweisen Kühlung der Ablationssonde zur Vermeidung einer Karbonisation des Gewebes umfasst, die durch eine Versorgungseinrichtung (60) gespeist werden kann,
wobei die Kühleinrichtung einen Fluidkanal im Inneren des Sondenschlauchs umfasst, wobei ein Fluid über den Fluidkanal in den distalen Bereich der Ablationssonde eingebracht werden kann, wo das Fluid an einem distalen Ende des Fluidkanals (23) expandieren kann, um der Ablationssonde (10) Wärmeenergie zu entziehen.

2. Ablationssonde nach Anspruch 1, **dadurch gekennzeichnet, dass** die Leitungsfolie (24) eine Gold-, Silber- oder Kupferfolie ist oder eine Legierung mit Gold- und/oder Silber- und/oder Kupfer-Komponenten umfasst.

3. Ablationssonde nach einem der vorhergehenden Ansprüche, insbesondere nach Anspruch 1, **dadurch gekennzeichnet, dass** benachbarte Bahnen der schraubenförmigen Struktur in Längsrichtung des Sondenschlauchs (21) voneinander beabstandet sind.

4. Ablationssonde nach einem der vorhergehenden Ansprüche, **gekennzeichnet durch** eine Isolatorschicht (26), die zur Fixierung der Leitungsfolie (24) diese zumindest abschnittsweise überdeckt.

5. Ablationssonde nach einem der vorhergehenden Ansprüche, **dadurch gekennzeichnet, dass** die mindestens eine Elektrode die Leitungsfolie (24) unmittelbar kontaktiert.

6. Ablationssonde nach einem der vorhergehenden Ansprüche, **dadurch gekennzeichnet, dass** der Fluidkanal (23) in einem Lumen (29) des Sondenschlauchs (21) angeordnet ist.

## Claims

1. Ablation probe (10), comprising:
- a probe hose (21) for transporting fluid,
- a distal electrode (30) and a proximal electrode (35) which, for devitalising tissue by means of HF current, are connected to a supply device (60) containing an HF generator, and
- conductive foils (24) arranged parallel to each other along a longitudinal axis (27) of the probe hose (21) and in a helical structure, wherein the distal electrode and the proximal electrode are electrically isolated from each other and each powered by means of a conductive foil (24), wherein the ablation probe (10) comprises a cooling device for cooling at least portions of the ablation probe to prevent carbonisation of the tissue, which device may be supplied by a supply device (60),
wherein the cooling device comprises a fluid channel in the interior of the probe hose, wherein a fluid can be brought via the fluid channel to the distal region of the ablation probe where the fluid can expand at a distal end of the fluid channel (23) in order to extract thermal energy from the ablation probe (10).

2. Ablation probe according to claim 1, **characterised in that** the conductive foil (24) is a gold, silver or copper foil or comprises an alloy with gold and/or silver and/or copper components.

3. Ablation probe according to any of the preceding claims, in particular claim 1, **characterised in that** adjacent tracks of the helical structure are spaced apart from each other in the longitudinal direction of the probe hose (21).

4. Ablation probe according to any of the preceding claims, **characterised by** an isolator layer (26) which, for fixing the conductive foil (24), covers this at least in portions.

5. Ablation probe according to any of the preceding claims, **characterised in that** the at least one electrode contacts the conductive foil (24) directly.

6. Ablation probe according to any of the preceding claims, **characterised in that** the fluid channel (23) is arranged in a lumen (29) of the probe hose (21).

## Revendications

1. Sonde d'ablation (10), comprenant :
- un tube de sonde souple (21) pour le transport de fluide ;
- une électrode distale (30) et une électrode proximale (35), qui, en vue de la dévitalisation de tissus au moyen d'un courant HF, sont reliées à un dispositif d'alimentation (60) contenant un générateur HF ; et
- des feuilles conductrices (24) qui sont disposées le long d'un axe longitudinal (27) du tube de sonde souple (21), parallèlement les unes aux autres et dans une structure hélicoïdale, l'électrode distale et l'électrode proximale étant isolées électriquement l'une vis-à-vis de l'autre et étant alimentées respectivement par l'intermédiaire d'une feuille conductrice (24), la sonde d'ablation (10) comprenant un dispositif de refroidissement destiné au refroidissement, au moins par portions, de la sonde d'ablation, aux fins d'éviter une carbonisation du tissu, qui peut être alimenté par un dispositif d'alimentation (60),
le dispositif de refroidissement comportant un conduit de fluide à l'intérieur du tube de sonde souple, un fluide pouvant être introduit via le conduit de fluide dans la partie distale de la sonde d'ablation, où le fluide peut se dilater à une extrémité distale du conduit de fluide (23), afin de prélever de l'énergie thermique sur la sonde d'ablation (10).

2. Sonde d'ablation selon la revendication 1, **caractérisée en ce que** la feuille conductrice (24) est une feuille d'or, d'argent ou de cuivre ou comprend un alliage avec des composants d'or et/ou d'argent et/ou de cuivre.

3. Sonde d'ablation selon l'une des revendications précédentes, en particulier selon la revendication 1, **caractérisée en ce que** des bandes adjacentes de la structure hélicoïdale sont espacées les unes des autres dans le sens longitudinal du tube de sonde souple (21).

4. Sonde d'ablation selon l'une des revendications précédentes, **caractérisée en ce qu'**elle comporte une couche d'isolateur (26) qui, en vue de la fixation de la feuille conductrice (24), recouvre celle-ci au moins par portions.

5. Sonde d'ablation selon l'une des revendications précédentes, **caractérisée en ce que** l'électrode, au nombre d'au moins une, est en contact direct avec la feuille conductrice (24).

6. Sonde d'ablation selon l'une des revendications précédentes, **caractérisée en ce que** le conduit de fluide (23) est disposé dans une lumière (29) du tube de sonde souple (21).
